# EUROPEAN PATENT APPLICATION

(11) **EP 4 293 118 A1**
(43) Date of publication of application: **20.12.2023**
(21) Application number: 22752808.0
(22) Date of filing: 10.02.2022
(51) Int. Cl.: C12N 15/864, C12N 5/10, C12N 7/01, C12N 15/35

(54) **KIT FOR PRODUCING ADENO-ASSOCIATED VIRUS, AND USE OF SAID KIT**

(30) Priority: 12.02.2021 JP 2021020868
(71) Applicant: FUJIFILM Corporation, Tokyo 106-8620 (JP)
(72) Inventor: MORI Yusuke, Ashigarakami-gun, Kanagawa 258-8577 (JP); MURAGUCHI Taichi, Ashigarakami-gun, Kanagawa 258-8577 (JP); UMETANI Sayako, Ashigarakami-gun, Kanagawa 258-8577 (JP); WATANABE Yuya, Ashigarakami-gun, Kanagawa 258-8577 (JP)
(74) Representative: Hoffmann Eitle
(86) International application number: PCT/JP2022/005335
(87) International publication number: WO 2022/172986

(57) **Abstract**

An object of the present invention is to provide a kit and an integration vector for preparing an adeno-associated virus-producing cell capable of controlled manufacture of an adeno-associated virus, and to provide an adeno-associated virus-producing cell capable of controlled manufacture of an adeno-associated virus, a manufacturing method of the same, and a manufacturing method of the adeno-associated virus. According to the present invention, a kit including a first vector containing one or more virus helper genes, in which at least one of the virus helper genes is under a control of a promoter capable of regulating expression, and a second vector containing one or more adeno-associated virus genes, in which at least one of the adeno-associated virus genes is not under a control of a promoter regulating expression is provided.

## Description

### Technical Field

The present invention relates to a kit and an integration vector for preparing adeno-associated virus-producing cells. Furthermore, the present invention relates to adeno-associated virus-producing cells, a manufacturing method of the same, and a manufacturing method of an adeno-associated virus.

### Background Art

The adeno-associated virus (also referred to as AAV) is a linear single-stranded DNA virus belonging to Parvoviridae. The wild-type AAV genome contains a replication regulatory gene (rep gene) and a capsid structural gene (cap gene), and inverted terminal repeat (ITR) sequences are adjacent to these genes for viral replication and packaging. The AAV vector can transfer a gene to proliferating and non-proliferating cells, and can be expressed for a long term particularly in non-dividing cells. In addition, AAV is considered to be non-pathogenic and has low immunogenicity. From the above, the AAV vector has been clinically applied as a vector for gene therapy.

AAV is a non-enveloped virus that proliferates in the presence of helper viruses such as adenovirus and herpesvirus. In the preparation of AAV to be used for gene therapy or nucleic acid transfer, classically, AAV replication has been performed by co-infecting host cells with an adenovirus. In addition, a gene responsible for the helper action of adenovirus has been clarified, and a plasmid containing this gene has also been used. For example, a plasmid containing a rep gene and a cap gene, an adenovirus helper plasmid, and a plasmid containing a transgene are simultaneously transfected into HEK293 cells, and can then be packaged as recombinant AAV (rAAV).

Patent Document 1describes that mammalian cells containing a nucleic acid molecule encoding a virus helper gene under the control of a first derepressible promoter, a nucleic acid molecule encoding an AAV gene under the control of a second derepressible promoter, and a nucleic acid encoding a repressor element of a first and second derepressible promoter, is used to produce AAV.

Patent Document 2 describes (a) a base sequence encoding a REP protein of AAV, (b) a base sequence encoding a capsid structural protein of AAV, (c) a base sequence containing a first inverted terminal repeat (ITR) of AAV, (d) a base sequence containing a second inverted terminal repeat (ITR) of AAV, (e) a base sequence for inserting a base sequence encoding a foreign protein and/or a base sequence encoding a foreign protein located between the first ITR and the second ITR, (f) a base sequence containing an E2A region of an adenovirus, (g) a base sequence containing an E4 region of an adenovirus, and (h) a nucleic acid molecule having a base sequence containing a VA1 RNA region of an adenovirus.

Patent Document 3 describes an adeno-associated virus (AAV) producer cell having nucleic acid sequences encoding an AAV rep/cap gene, a helper virus gene, and a DNA genome of an AAV vector particle, in which the nucleic acid sequences are all integrated together into a single locus in the genome of the AAV producer cell.

### Prior Art Documents

### Patent Documents

Patent Document 1:WO2020-132059A
Patent Document 2: JP2020-188763A
Patent Document 3 JP2020-517238A

### SUMMARY OF THE INVENTION

In the AAV-producing cells described in Patent Document 1, it is considered that the cell disorder that occurs at the time of establishment of the AAV-producing cell strain is avoided by artificial sequence addition to the gene. However, in the AAV-producing cells described in Patent Document 1, since artificial control sequences are added to each of the virus helper gene and the AAV gene, there is a possibility that crosstalk between control sequences occurs (it becomes difficult to control the expression level for each target gene by mutual interference between control sequences), and there is a possibility that AAV production cannot be sufficiently controlled. In the AAV-producing cells described in Patent Documents 2 and 3, there is no specific description about controlling expression of a virus helper gene by a promoter capable of regulating the expression.

An object of the present invention is to provide a kit and an integration vector for preparing an adeno-associated virus-producing cell capable of controlled manufacture of an adeno-associated virus. Furthermore, an object of the present invention is to provide an adeno-associated virus-producing cell capable of controlled manufacture of an adeno-associated virus, a manufacturing method of the same, and a manufacturing method of the adeno-associated virus.

As a result of diligent studies to solve the above objects, the present inventors have found that AAV-producing cells capable of controlled manufacture of an adeno-associated virus by using a first vector containing at least one virus helper gene under the control of a promoter capable of regulating the expression, and a second vector containing at least one adeno-associated virus gene not under the control of a promoter regulating expression by a drug. The present invention has been completed based on the above findings.

According to an aspect of the present invention, the following inventions are provided.
<1> A kit including
   a first vector containing one or more virus helper genes, in which at least one of the virus helper genes is under a control of a promoter capable of regulating expression, and
   a second vector containing one or more adeno-associated virus genes, in which at least one of the adeno-associated virus genes is not under a control of a promoter regulating expression by a drug.
<2> The kit according to <1>, in which the promoter capable of regulating expression in the first vector is a promoter capable of regulating expression by a drug.
<3> The kit according to <1> or <2>, in which the virus helper gene contained in the first vector is derived from adenovirus.
<4> The kit according to <1> or <2>, in which among the virus helper genes contained in the first vector, an E4 gene and a VA-RNA gene are under a control of a promoter capable of regulating expression.
<5> The kit according to any one of <1> to <4>, in which all of the virus helper genes contained in the first vector are under a control of a promoter capable of regulating expression.
<6> The kit according to any one of <1> to <5>, in which the virus helper genes contained in the first vector are an E2 gene, an E4 gene, and a VA-RNA gene.
<7> The kit according to <6>, in which the E4 gene is an E4ORF6 gene.
<8> The kit according to <6> or <7>, in which the VA-RNA gene is a VA-RNAI gene.
<9> The kit according to any one of <1> to <8>, in which in the second vector, all of the adeno-associated virus genes are not under a control of a promoter regulating expression by a drug.
<10> The kit according to any one of <1> to <9>, in which in the second vector, any one of the adeno-associated virus genes, which is not under a control of a promoter regulating expression by a drug is under a control of a promoter that is naturally carried by the adeno-associated virus gene.
<11> The kit according to any one of <1> to <10>, in which the adeno-associated virus genes contained in the second vector are a rep gene and a cap gene.
<12> The kit according to any one of <1> to <11>, in which among the adeno-associated virus genes contained in the second vector, the adeno-associated virus gene that is not under a control of a promoter regulating expression by a drug is a rep gene.
<13> A cell including the first vector and the second vector which are included in the kit according to any one of <1> to <12>.
<14> The cell according to <13>, in which the cell is a human cell.
<15> The cell according to <13> or <14>, in which the first vector and the second vector are contained in a chromosome.
<16> An integration vector including, in a connected state,
   a first vector containing one or more virus helper genes, in which at least one of the virus helper genes is under a control of a promoter capable of regulating expression, and
   a second vector containing one or more adeno-associated virus genes, in which at least one of the adeno-associated virus genes is not under a control of a promoter regulating expression by a drug.
<17> The integration vector according to <16>, further including a third vector containing a gene for desired treatment or prevention.
< 18> A cell having the integration vector according to < 16> or < 17>.
<19> The cell according to <18>, in which the cell is a human cell.
<20> The cell according to <18> or <19>, in which the integration vector is contained in a chromosome.
<21> A manufacturing method of a cell for producing an adeno-associated virus including transferring, into a cell, a first vector containing one or more virus helper genes, in which at least one of the virus helper genes is under a control of a promoter capable of regulating expression, and a second vector containing one or more adeno-associated virus genes, in which at least one of the adeno-associated virus genes is not under a control of a promoter regulating expression.
<22> A manufacturing method of an adeno-associated virus, including
   a step of culturing cells that contains a first vector containing one or more virus helper genes, in which at least one of the virus helper genes is under a control of a promoter capable of regulating expression, a second vector containing one or more adeno-associated virus genes, in which at least one of the adeno-associated virus genes is not under a control of a promoter regulating expression, and as necessary, a third vector containing a gene for desired treatment or prevention, and
   a step of expressing the virus helper gene under the control of the promoter capable of regulating expression by activating the promoter capable of regulating expression.
<23> A manufacturing method of an adeno-associated virus, including
   a first step of transferring a part of a virus helper gene and a part of an adeno-associated virus gene into a cell,
   a second step of culturing and proliferating the cell obtained in the first step,
   a third step of transferring a virus helper gene not contained in the cell obtained in the first step, an adeno-associated virus gene not contained in the cell obtained in the first step, and as necessary, a gene for desired treatment or prevention, into the cells obtained in the second step, and
   a fourth step of culturing the cells obtained in the third step.
<24> The manufacturing method of an adeno-associated virus according to <23>, in which the virus helper gene transferred into the cell in the first step is an E2 gene, the adeno-associated virus gene transferred into the cell in the first step is a rep gene, the virus helper genes transferred into the cells in the third step are an E4 gene and a VA-RNA gene, and the adeno-associated virus gene transferred into the cells in the third step is a cap gene.
<25> A manufacturing method of an adeno-associated virus, including
   a first step of transferring a part of a virus helper gene, an adeno-associated virus gene, and as necessary, a gene for desired treatment or prevention, into a cell,
   a second step of culturing and proliferating the cell obtained in the first step,
   a third step of transferring a virus helper gene not contained in the cell in the first step into the cells obtained in the second step, and
   a fourth step of culturing the cells obtained in the third step.
<26> The manufacturing method of an adeno-associated virus according to <25>, in which the virus helper gene transferred into the cell in the first step is an E2 gene, the adeno-associated virus genes transferred into the cell in the first step are a rep gene and a cap gene, and the virus helper genes transferred into the cells in the third step are an E4 gene and a VA-RNA gene.
<27> A manufacturing method of an adeno-associated virus, including
   a first step of transferring an adeno-associated virus gene into a cell,
   a second step of culturing and proliferating the cell obtained in the first step,
   a third step of transferring a virus helper gene and as necessary, a gene for desired treatment or prevention into the cells obtained in the second step, and
   a fourth step of culturing the cells obtained in the third step.
<28> The manufacturing method of an adeno-associated virus according to <27>, in which the adeno-associated virus genes transferred into the cell in the first step are a rep gene and a cap gene, and the virus helper genes transferred into the cells in the third step are an E2 gene, an E4 gene, and a VA-RNA gene.
<29> A manufacturing method of an adeno-associated virus, including
   a first step of transferring a part of a virus helper gene and as necessary, a gene for desired treatment or prevention, into a cell,
   a second step of culturing and proliferating the cell obtained in the first step,
   a third step of transferring a virus helper gene not contained in the cell in the first step and an adeno-associated virus gene into the cells obtained in the second step, and
   a fourth step of culturing the cells obtained in the third step.
<30> The manufacturing method of an adeno-associated virus according to <29>, in which the virus helper gene transferred into the cell in the first step is an E2 gene, the virus helper genes transferred into the cells in the third step are an E4 gene and a VA-RNA gene, and the adeno-associated virus genes transferred into the cells in the third step is a rep gene and a cap gene.

According to an aspect of the present invention, there is provided an adeno-associated virus-producing cell capable of controlled manufacture of an adeno-associated virus. According to an aspect of the present invention, the adeno-associated virus can be manufactured in a controlled manner.

### BRIEF DESCRIPTION OF THE DRAWINGS

Fig. 1 shows the results of transferring genes into HEK293 cells in a predetermined combination and evaluating cytotoxicity.
Fig. 2 shows the results of transferring genes into HEK293 cells in a predetermined combination and evaluating protein expression.
Fig. 3 shows the results of transferring genes into HEK293 cells in a predetermined combination and evaluating cytotoxicity.
Fig. 4 shows the results of transferring genes into HEK293 cells in a predetermined combination and evaluating the AAV titer.
Fig. 5 shows the results of transferring genes into HEK293 cells in a predetermined combination and evaluating the AAV titer.
Fig. 6 shows the results of transferring genes into HEK293 cells in a predetermined combination and evaluating the AAV titer.
Fig. 7 shows the results of transferring genes into HEK293 cells in a predetermined combination and evaluating the AAV titer.
Fig. 8 shows the results of transferring genes into HEK293 cells in a predetermined combination and evaluating the AAV titer.
Fig. 9 shows the results of transferring genes into HEK293 cells in a predetermined combination and evaluating the AAV titer.
Fig. 10 shows the results of evaluation of AAV titer of an AAV producer cell strain in which genes have been transferred into HEK293 cells in a predetermined combination and a plasmid was inserted into the genome.
Fig. 11 shows a plasmid map of seq43.

### DESCRIPTION OF THE PREFERRED EMBODIMENTS

Hereinafter, an example of the embodiment of the present disclosure will be described. However, the present disclosure is not limited to the following embodiments, and can be implemented with appropriate changes within the scope of the object of the present disclosure. In the present specification, a numerical range indicated using "to" means a range including numerical values described before and after "to" as a minimum value and a maximum value, respectively.

In the present invention, in a case where the wild-type gene is modified by substitution, deletion, insertion, addition, or the like of a base, the number of the modified bases is preferably 1 to 20, more preferably 1 to 10, and still more preferably 1 to 3. The base sequence of the modified gene exhibits a sequence identity of preferably 85% or more, more preferably 90% or more, still more preferably 95%, and even still more preferably 98% or more with the base sequence of the wild-type gene.

### <Kit>

The kit according to the embodiment of the present invention includes
a first vector containing one or more virus helper genes, in which at least one of the virus helper genes is under the control of a promoter capable of regulating expression, and
a second vector containing one or more adeno-associated virus genes, in which at least one of the adeno-associated virus genes is not under the control of a promoter regulating expression by a drug.

For example, the kit according to the embodiment of the present invention is a first vector containing one or more virus helper genes, in which among the virus helper genes contained in the first vector, only the E4 gene and the VA-RNA gene are under the control of a promoter capable of regulating expression, and
a second vector containing one or more adeno-associated virus genes, in which among the adeno-associated virus genes contained in the second vector, the adeno-associated virus gene that is not under the control of a promoter regulating expression by a drug is a rep gene.

The kit according to the embodiment of the present invention is used for manufacturing AAV, may include a combination of vector sequences including a first vector and a second vector, and may be in a state dissolved or dispersed in a buffer composition, purified water, or the like. The kit may be combined with a composition useful for implementing the present invention, such as a reagent that transfers a vector, or may contain a suitable container, for example, a vial, a tube, a test tube, or another container.

In the method described in WO2020-132059A, sequences for controlling expression are artificially added to a plurality of positions of a helper gene and an adeno-associated virus gene, however, a crosstalk by using a plurality of artificial sequences has been a problem. In the present invention, the above-described problem of crosstalk is solved by limiting the use of a promoter capable of regulating expression.

In the present invention, it has been revealed that the expression of an AAV gene known to have a cell disorder depends on the helper gene, and therefore it turned out that the cell disorder can be avoided by controlling only the expression of the helper gene. That is, in the present invention, the cell disorder that occurs at the time of establishment of the AAV-producing cell strain can be avoided by adding a minimum artificial sequence. The cell disorder can be evaluated by measuring the viability of the cells after the gene transfer, for example, by trypan blue staining. In addition, in order to eliminate concerns such as crosstalk caused by increasing the number of artificial sequences, natural sequences are used as much as possible in the present invention, whereby a production system that mimics the AAV production mechanism in the natural world has been built.

Furthermore, in the conventional AAV manufacturing method, it is necessary to transfer an AAV-producing gene at each production, and the suitability for scale-up is poor from the viewpoint of a cost and a step. By using the AAV-producing cell according to the embodiment of the present invention, it is possible to suppress a cost and a step of gene transfer and to prepare a large amount of AAV required for treatment.

In the present specification, a promoter means a DNA regulatory region/sequence to which an RNA polymerase can be bound and which is involved in the initiation of transcription of a coding sequence or a non-coding sequence.

In the present specification, a vector is a DNA or RNA molecule that is used to artificially convey a foreign genetic material to another cell. In a case where a vector containing a foreign genetic material to be transferred is transferred into a cell, the foreign genetic material is replicated and/or expressed in the cell. Examples of the vector include an episomal (for example, plasmid) vector and a non-episomal vector. The vector can be transferred into a host cell by methods such as transfection, transduction, cell fusion, and lipofection.

In the present specification, a promoter naturally carried by a certain gene means a promoter known to express the gene in a natural state, not artificially.

### (First vector)

The first vector contains one or more virus helper genes, and at least one of the virus helper genes is under the control of a promoter capable of regulating expression.

As the promoter capable of regulating expression, a promoter capable of regulating the on and off of expression depending on the presence or absence of a stimulus can be used. Examples of the stimulus include chemical stimuli (intrinsic hormone/stress response, lactose, tetracycline or a derivative thereof (for example, doxycycline), cumic acid, a protein (rapamycin, FKCsA, abscisic acid (ABA), and the like), tamoxifen/Cre-loxP (a system using a Cre promoter modified such that activation can be induced by tamoxifen), and a riboswitch), and a physical stimulus (blue light, heat), but the stimulus is not particularly limited.

Preferably, the promoter capable of regulating expression in the first vector is a promoter capable of regulating expression by a drug. The drug is preferably tetracycline or a derivative thereof (for example, doxycycline).

Examples of the promoter capable of regulating expression include a tetracycline-responsive promoter, a RU486-inductive promoter, an ecdysone-inductive promoter, a rapamycin-inductive promoter, and a metallothionein promoter. Specific examples of the tetracycline-responsive promoter include a Tet on/off system (manufactured by TET systems GmbH & Co. KG).

The promoter capable of regulating expression in the first vector is particularly preferably a Tet on/off system that is a promoter capable of regulating expression by tetracycline and most preferably a Tet on system. The promoter is not particularly limited, but examples thereof include a cytomegalovirus-derived promoter (including an enhancer as desired), an SV40 early promoter, a human elongation factor-1α (EF-1α) promoter, a human ubiquitin C promoter, a retrovirus Rous sarcoma virus LTR promoter, a dihydrofolate reductase promoter, a β-actin promoter, a phosphoglycerate kinase (PGK) promoter, an H1 promoter (RNA Polymerase III promoter), and a U6 promoter (RNA Polymerase III promoter).

The promoter used in the Tet on system for regulating VA-RNA gene expression may be an H1 promoter (RNA Polymerase III promoter).

In the Tet on system, the promoter additionally contains at least one Tet operon. By using a Tet operon (tetracycline-controlled transcriptional activation), transcription can be reversibly switched on or off in the presence of one of the tetracycline which is an antibiotic substance, or a derivative thereof (for example, doxycycline). The Tet repressor protein present in the cell blocks expression by binding to a Tet operator sequence introduced into a promoter. Therefore, no gene expression is observed in a case where the Tet repressor binds to the Tet operator sequence. By adding tetracycline or doxycycline, the Tet repressor is sequestered to allow promoter activity and turn on gene expression. The Tet operon system is widely available, such as the Tet operon used in the pcDNA (trademark) 4/TO mammalian expression vector available from Invitrogen.

In the Tet-on system, the Tet operator sequence may be located upstream or downstream of the promoter for which expression is desired to be regulated. The Tet operator sequence is preferably located downstream of a promoter capable of regulating expression in view of reducing expression leakage in a case where expression is turned off.

The virus helper gene is a non-adeno-associated virus gene for enabling replication and packaging of an adeno-associated virus. As the virus helper gene, a gene derived from a virus of another species other than the adeno-associated virus is used. Specific examples of the virus helper gene include a virus helper gene derived from an adenovirus or a herpesvirus, and the virus helper gene is preferably derived from an adenovirus.

The adenovirus refers to a non-enveloped virus having an icosahedron nucleocapsid containing double-stranded DNA in a family of Adenoviridae. Over 50 subtypes of adenovirus have been isolated from humans and many additional subtypes have been isolated from other mammals and birds. These subtypes belong to the family of Adenoviridae and are classified into two genera (that is, Mastadenovirus and Aviadenovirus). These adenoviruses are morphologically and structurally similar. However, in humans, adenoviruses exhibit different immunological properties, and that is, are classified into serum types. Two human serum types of adenovirus (that is, Ad2 and Ad5) have been intensively studied.

The adenovirus-derived virus helper gene is a gene involved in replication and packaging of adeno-associated virus. Examples of the adenovirus-derived virus helper gene include an E1A gene, an E1B gene, an E2A gene, an E4 gene, and a VA-RNA gene.

The virus helper gene contained in the first vector is preferably an E2 gene, an E4 gene, and a VA-RNA gene.

The virus helper gene contained in the first vector is more preferably an E4 gene and a VA-RNA gene.

The E4 gene is preferably the E4ORF6 gene.

The VA-RNA gene is preferably a VA-RNAI gene.

The adenovirus-derived virus helper gene (such as the E1A gene, the E1B gene, the E2A gene, the E4 gene, and the VA-RNA gene) may be a wild-type gene, but a gene in which modifications such as substitution, deletion, insertion, or addition of a base have been made to the wild-type gene may be used as long as it exhibits an inherent function.

In a case where modifications such as substitution, deletion, insertion, or addition of a base is made to the wild-type virus helper gene, the number of the modified bases is preferably 1 to 20, more preferably 1 to 10, and still more preferably 1 to 3. The modified base sequence of the virus helper gene exhibits preferably 85% or more sequence identity, more preferably 90% or more sequence identity, still more preferably 95% or more sequence identity, and even still more preferably 98% or more sequence identity with the base sequence of the wild-type virus helper gene.

In a case where the first vector contains the E4 gene and the VA-RNA gene, the arrangement thereof is not particularly limited, and the E4 gene may be located upstream of the VA-RNA gene or downstream of the VA-RNA gene, and the E4 gene is located preferably upstream of the VA-RNA gene.

The virus helper gene contained in the first vector is under the control of a promoter capable of regulating expression.

It is preferable that among the virus helper genes contained in the first vector, the E2 gene is not under the control of a promoter capable of regulating expression.

Among the virus helper genes contained in the first vector, only the E4 gene may be under the control of a promoter capable of regulating expression. Among the virus helper genes contained in the first vector, only the VA-RNA gene may be under the control of a promoter capable of regulating expression. It is preferable that among the virus helper genes contained in the first vector, the E4 gene and the VA-RNA gene are under the control of a promoter capable of regulating expression.

The first vector is not limited as long as it contains a nucleic acid, and for example, a plasmid vector, an artificial chromosome vector, a virus vector, a vector having an artificially designed nucleic acid, or the like can be used, and the plasmid vector is preferable.

### (Second vector)

The second vector contains one or more adeno-associated virus genes, and at least one of the above-mentioned adeno-associated virus genes is not under the control of a promoter regulating expression by a drug.

Adeno-associated virus (AAV) refers to a small, incompletely replicative, non-enveloped, non-enveloped virus containing single-stranded DNA from the family of Parvoviridae and Dependoparvovirus. It is known that there are more than 100 serum types in AAV, and that the host range and the characteristics of the virus differ depending on the difference in the serum types. Serum type 2 (AAV2) is one of the serum types that have been widely studied for a long time, and it is known that the host range is very wide. The serum type 1 (AAV1), the serum type 5 (AAV5), and the serum type 6 (AAV6) are serum types having higher tissue directivity. It is said that AAV1 has high gene transfer efficiency into muscles, liver, airway, central nervous system, and the like, AAV5 has high gene transfer efficiency into central nervous system, liver, retina, and the like, and AAV6 has high gene transfer efficiency into heart, muscle, liver, and the like. In the present invention, the serum type 2 or the serum type 5 is preferably used. The serum type 5 is particularly preferable.

The adeno-associated virus gene refers to a gene composed of one or a plurality of nucleic acid sequences derived from the serum types of one or a plurality of adeno-associated viruses. The adeno-associated virus gene is preferably a gene involved in replication and packaging of AAV and a gene encoding an AAV constituent protein.

In the second vector, any one of all adeno-associated virus genes is preferably not under the control of a promoter regulating expression by a drug.

In the second vector, any one of the adeno-associated virus genes that is not under the control of a promoter regulating expression by a drug is preferably under the control of a promoter, and more preferably under the control of a promoter that is naturally carried by the adeno-associated virus gene.

In the second vector, all adeno-associated virus genes are preferably not under the control of a promoter regulating expression, and all adeno-associated virus genes are more preferably under the control of a promoter that is naturally carried.

Examples of the promoter that is naturally carried by the rep gene can include p5 and p19 promoters. Examples of the promoter that is naturally carried by the cap gene can include a p40 promoter.

The adeno-associated virus gene contained in the second vector is preferably a rep gene and a cap gene.

The adeno-associated virus gene (such as a rep gene and a cap gene) may be a wild-type gene, but a gene in which modifications such as substitution, deletion, insertion, or addition of a base have been made to the wild-type gene may be used as long as it exhibits an inherent function.

In a case where modifications such as substitution, deletion, insertion, or addition of a base is made to the wild-type adeno-associated virus gene (such as a rep gene and a cap gene), the number of the modified bases is preferably 1 to 20, more preferably 1 to 10, and still more preferably 1 to 3. The modified base sequence of the adeno-associated virus gene exhibits preferably 85% or more sequence identity, more preferably 90% or more sequence identity, still more preferably 95% or more sequence identity, and even still more preferably 98% or more sequence identity with the base sequence of the wild-type adeno-associated virus gene.

In a case where the second vector contains the rep gene and the cap gene, the arrangement thereof is not particularly limited, and the rep gene may be located upstream of the cap gene or downstream of the cap gene, and the rep gene is located preferably upstream of the cap gene.

The rep gene means a region of the AAV genome encoding a virus replication protein that is known to those skilled in the art and that is collectively required for replication of a virus genome, or its functional homolog, for example, a human herpesvirus 6 (HHV-6) rep gene (mediating AAV-2 DNA replication is known). Accordingly, the coding region of the rep gene includes at least a gene encoding REP78 and REP68 ("long form of REP protein") and REP52 and REP40 ("short form of REP protein") of AAV, or a functional homologue thereof. The coding region of the rep gene used in the present invention may be derived from any AAV serum type, but is preferably derived from AAV2. Examples of those derived from AAV2 include REP78 and REP68, and REP52 and REP40, and ITR.

The cap gene means a region in the AAV genome encoding a capsid protein of a virus known to those skilled in the art. Examples of these capsid proteins are AAV capsid proteins VP1, VP2 and VP3. The cap gene used in the present invention may be derived from any AAV serum type, but is preferably derived from AAV2 or AAV5. AAV5 is particularly preferable.

Among the adeno-associated virus genes contained in the second vector, the adeno-associated virus gene that is not under a control of a promoter regulating expression by a drug is preferably a rep gene.

The second vector is not limited as long as it contains a nucleic acid, and for example, a plasmid vector, an artificial chromosome vector, a virus vector, a vector having an artificially designed nucleic acid, or the like can be used, and the plasmid vector is preferable.

### <Integration vector>

Furthermore, the present invention relates to the integration vector having a kit including a first vector containing one or more virus helper genes, in which at least one of the virus helper genes is under a control of a promoter capable of regulating expression, and a second vector containing one or more adeno-associated virus genes, in which at least one of the adeno-associated virus genes is not under a control of a promoter regulating expression, in a connected state. The connected state refers to a state in which the vectors are directly linked or indirectly linked by another sequence. In a case where the first vector or the second vector is cyclic, a vector obtained by cleaving a part of the sequence of the vector to make it linear and linking can be used as an integration vector.

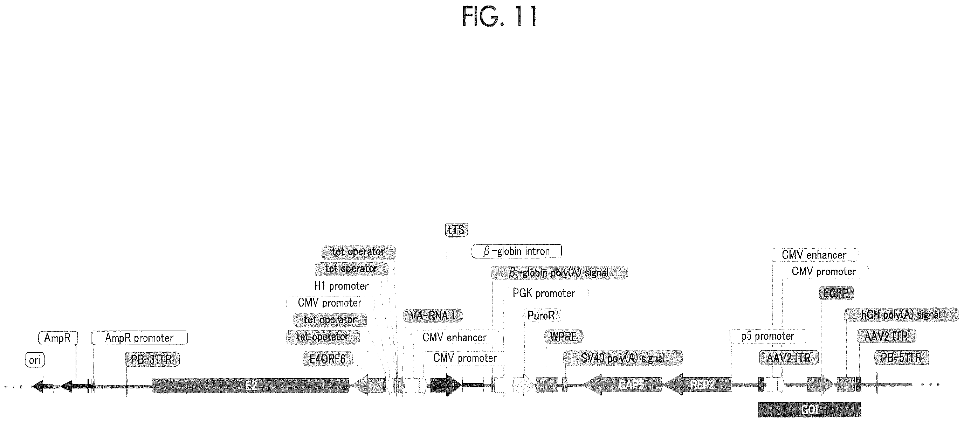

The integration vector according to the embodiment of the present invention may be a vector in which the entire base sequence is synthesized as in seq43 whose structure is shown in Fig. 11 used in Examples. In the vector shown in Fig. 11, the E4 gene and its upstream promoter, and the VA-RNA gene and its upstream promoter correspond to the first vector, the rep gene and the cap gene, and their upstream promoter correspond to the second vector. In the integration vector shown in Fig. 11, the first vector and the second vector are connected to each other.

The integration vector is not limited as long as it contains a nucleic acid, and for example, a plasmid vector, an artificial chromosome vector, a virus vector, or the like can be used, and the plasmid vector is preferable.

The configurations of the first vector and the second vector in the integration vector are as described in the above-described <kit> in the present specification.

The integration vector according to the embodiment of the present invention may further include a third vector containing a gene for desired treatment or prevention. In a case where the third vector is cyclic, a vector obtained by cleaving a part of the sequence of the vector to make it linear and linking can be used as an integration vector.

The third vector is a vector containing a gene for desired treatment or prevention.

As the gene for treatment or prevention, a gene that is incomplete or lost in the genome of the target cell, a gene encoding non-natural protein having a desired biological or therapeutic effect (for example, antiviral function), or the like can be used, but the gene is not particularly limited. Specific examples of the gene for treatment or prevention include a gene used for treatment or prevention of inflammatory diseases, autoimmunity, chronic and infectious diseases (including disorders such as AIDS, cancer, nervous system diseases, cardiovascular diseases, and hypercholesterolemia), various blood diseases such as anemia and hemophilia, or gene deficiency (for example, cystic fibrosis, Gaucher's disease, adenosine deaminase (ADA) deficiency, emphysema, and the like).

The gene for treatment or prevention may be several antisense oligonucleotides (for example, a short-chain oligonucleotide complementary to a sequence around a translation initiation site (AUG codon) of mRNA) that are useful in antisense therapy against cancer and viral disease.

The third vector may include a promoter for expressing a gene for treatment or prevention. The promoter for expressing a gene for treatment or prevention is not particularly limited, but examples thereof can include a cytomegalovirus-derived promoter (including an enhancer as desired), an SV40 early promoter, a human elongation factor-1α (EF-1α) promoter, a human ubiquitin C promoter, a retrovirus Rous sarcoma virus LTR promoter, a dihydrofolate reductase promoter, a β-actin promoter, and a phosphoglycerate kinase (PGK) promoter. The gene for treatment or prevention and the promoter for expressing the gene are preferably flanked by the ITR sequences.

The third vector is not limited as long as it contains a nucleic acid, and for example, a plasmid, an artificial chromosome, a virus, or the like can be used, and the plasmid is preferable.

### <Cell>

The present invention relates to a cell having the first vector and the second vector described above. The present invention further relates to a cell having the above-mentioned integration vector. The present invention further relates to a cell having a first vector, a second vector, and a third vector.

The cell is preferably a eukaryotic cell and more preferably a mammalian cell. Examples of the mammalian cell include a human cell, a mouse cell, a rat cell, a monkey cell, and a hamster cell, but the cells are not particularly limited. The human cell can be preferably used. Examples of the cell include mouse myeloma (NSO) cell lines, Chinese hamster ovary (CHO) cell lines, HT1080, H9, HepG2, MCF7, MDBK Jurkat, NIH3T3, PC12, a baby hamster kidney (BHK) cell, VERO, SP2/0, YB2/0, Y0, C127, an L cell, COS (for example, COS1 and COS7), QC1-3, a human embryo-derived kidney (HEK293) cell, VERO, PER. C6, HeLa, EB1, EB2, EB3, and oncolytic or hybridoma cell lines. The cell is preferably a HEK293 cell or a CHO cell, and more preferably a HEK293 cell.

The cell according to the embodiment of the present invention preferably contains a first vector and a second vector, or an integration vector. In addition, the first vector, the second vector, and the third vector are preferably contained in a chromosome. Being contained in a chromosome means that the whole length or a part of the vector is integrated into the chromosome. Integration into the chromosome can be confirmed by performing genome sequence analysis and confirming that the sequence in the vector is bound to the host genome. Since the vector is integrated into the chromosome, the gene required for the production of the adeno-associated virus is constitutively maintained in the cell, and the adeno-associated virus can be produced at an optional timing.

In the cell according to the embodiment of the present invention, after the first vector and the second vector, the integration vector, or the first vector and the second vector and the third vector are transferred into the cell, the gene required for the production of the adeno-associated virus among the vectors transferred can be retained for one week or more. Therefore, it is possible to produce the adeno-associated vector after culturing the cells into which the vector has been transferred for one week or more. The adeno-associated vector can be produced after culturing preferably for 2 weeks or more, and the adeno-associated vector can be produced after culturing more preferably for 1 month or more.

### <Manufacturing method of cell for producing adeno-associated virus>

According to the present invention, a manufacturing method of a cell for producing an adeno-associated virus including transferring a first vector containing one or more virus helper genes, in which at least one of the virus helper genes is under a control of a promoter capable of regulating expression, and a second vector containing one or more adeno-associated virus genes, in which at least one of the adeno-associated virus genes is not under a control of a promoter regulating expression, into a cell is provided. A manufacturing method of a cell for producing an adeno-associated virus, further containing a third vector is provided. The configurations of the first vector, the second vector, and the third vector are as described in the above-described <kit> in the present specification. It is sufficient that the first vector, the second vector, and the third vector are transferred to be expressed, but it is more preferable to be a permanent expression. Permanent expression means that in a case where a cell divides, the first vector, the second vector, and the third vector are also replicated, and the divided cells also have the first vector, the second vector, and the third vector. Permanent expression can be achieved by integrating the vector into the chromosome of the cell or by using a vector capable of replicating in the cytoplasm.

The first vector and the second vector can be transferred into a cell by methods such as transfection, transduction, and lipofection.

Transfection refers to transfer of a nucleic acid into a cell across a membrane of eukaryotic cells by chemical means (for example, calcium phosphate mediated precipitation), mechanical means (for example, electroporation), or physical means (for example, delivery by bioballistic).

Transduction refers to transfer of a nucleic acid into a cell across a membrane of a eukaryotic cell via a virus-derived vector.

Lipofection refers to transfer of a nucleic acid into a cell by the formation of a complex between a vector and a positively charged lipid or the like by electrical interaction, and endocytosis or membrane fusion.

### <Manufacturing method of adeno-associated virus>

An example of the manufacturing method of an adeno-associated virus according to the embodiment of the present invention includes a method including
a step of culturing cells that contains a first vector containing one or more virus helper genes, at least one of the virus helper genes is under a control of a promoter capable of regulating expression, a second vector containing one or more adeno-associated virus genes, in which at least one of the adeno-associated virus genes is not under a control of a promoter regulating expression, and as necessary, a third vector containing a gene for desired treatment or prevention, and
a step of expressing the virus helper gene under the control of the promoter capable of regulating expression by activating the promoter capable of regulating expression.

The configurations of the first vector, the second vector, and the third vector are as described above in the present specification. The same applies to the cells as described above in the present specification.

To activate the promoter capable of regulating expression, for example, in a case where a promoter capable of regulating the on and off of expression by a stimulus is used, expression can be regulated on and off by applying the stimulus or excluding the stimulus.

Another example of the manufacturing method of an adeno-associated virus according to the embodiment of the present invention includes a method including
a first step of transferring a part of a virus helper gene and a part of an adeno-associated virus gene into a cell,
a second step of culturing and proliferating the cell obtained in the first step,
a third step of transferring a virus helper gene not contained in the cell obtained in the first step, an adeno-associated virus gene not contained in the cell obtained in the first step, and as necessary, a gene for desired treatment or prevention, into the cells obtained in the second step, and
a fourth step of culturing the cells obtained in the third step.

Details of the virus helper gene, the adeno-associated virus gene, and the gene for treatment or prevention are as described above in the present specification. In addition, the method for transferring a gene into a cell is also as described above in the present specification.

Preferably, the virus helper gene transferred into the cell in the first step is an E2 gene, the adeno-associated virus gene transferred into the cell in the first step is a rep gene, the virus helper genes transferred into the cells in the third step are an E4 gene and a VA-RNA gene, and the adeno-associated virus gene transferred into the cells in the third step is a cap gene, and more preferably, the gene for desired treatment or prevention is further transferred in the third step.

Yet another example of the manufacturing method of an adeno-associated virus according to the embodiment of the present invention includes a method including
a first step of transferring a part of a virus helper gene, an adeno-associated virus gene, and as necessary, a gene for desired treatment and prevention, into a cell,
a second step of culturing and proliferating the cell obtained in the first step,
a third step of transferring a virus helper gene not contained in the cell in the first step into the cells obtained in the second step, and
a fourth step of culturing the cells obtained in the third step.

Details of the virus helper gene, the adeno-associated virus gene, and the gene for treatment or prevention are as described above in the present specification. In addition, the method for transferring a nuclear gene into a cell is also as described above in the present specification.

In the second step, the cell doubling time may be within 200 hours, preferably within 100 hours, more preferably within 50 hours, and particularly preferably within 30 hours.

In the second step, the cell viability may be 65% or more, preferably 80% or more, and more preferably 95% or more.

Preferably, the virus helper gene transferred into the cell in the first step is an E2 gene, the adeno-associated virus genes transferred into the cell in the first step are a rep gene and a cap gene, and the virus helper genes transferred into the cells in the third step are an E4 gene and a VA-RNA gene.

Yet another example of the manufacturing method of an adeno-associated virus according to the embodiment of the present invention includes a method including
a first step of transferring an adeno-associated virus gene into a cell,
a second step of culturing and proliferating the cell obtained in the first step,
a third step of transferring a virus helper gene and as necessary, a gene for desired treatment or prevention into the cells obtained in the second step, and
a fourth step of culturing the cells obtained in the third step.

Details of the virus helper gene, the adeno-associated virus gene, and the gene for treatment or prevention are as described above in the present specification. In addition, the method for transferring a nuclear gene into a cell is also as described above in the present specification.

Preferably, the adeno-associated virus genes transferred into the cell in the first step are a rep gene and a cap gene, and the virus helper genes transferred into the cells in the third step are an E2 gene, an E4 gene, and a VA-RNA gene.

Yet another example of the manufacturing method of an adeno-associated virus according to the embodiment of the present invention includes a method including
a first step of transferring a part of a virus helper gene and as necessary, a gene for desired treatment and prevention, into a cell,
a second step of culturing and proliferating the cell obtained in the first step,
a third step of transferring a virus helper gene not contained in the cell in the first step and an adeno-associated virus gene into the cells obtained in the second step, and
a fourth step of culturing the cells obtained in the third step.

Details of the virus helper gene, the adeno-associated virus gene, and the gene for treatment or prevention are as described above in the present specification. In addition, the method for transferring a nuclear gene into a cell is also as described above in the present specification.

Preferably, the virus helper gene transferred into the cell in the first step is an E2 gene, the virus helper genes transferred into the cells in the third step are an E4 gene and a VA-RNA gene, and the adeno-associated virus genes transferred into the cells in the third step is a rep gene and a cap gene.

The cell culture in the manufacturing method of an adeno-associated virus according to the embodiment of the present invention can be carried out under normal conditions for cell culture. The culture medium to be used and the culture conditions can be appropriately selected by those skilled in the art. As the culture medium, the medium includes Expi29 Expression Medium (Thermo Fisher Scientific, A1435101), a Dulbecco's Modified Eagle's Medium (DMEM) containing 10% (vol/vol) bovine fetal serum (FBS), and a serum-free UltraCULTURE (trademark) medium (Lonza) can be used, but the present invention is not particularly limited.

The culture temperature is generally 25°C to 45°C, preferably 30°C to 42°C, more preferably 35°C to 40°C, and 37°C as an example.

The CO₂ concentration is generally 3 to 10% CO₂, preferably 5 to 10% CO₂, and 8% CO₂ as an example.

The cells can be cultured in a culture medium of any volume, for example, the cells can be cultured in a culture medium of 1 mL to 2,000 L, preferably 1 L to 2,000 L, more preferably 50 L to 2,000 L, and particularly preferably 500 L to 2,000 L.

The culture may be carried out while stirring by shaking. The stirring speed in the case of stirring by shaking is generally 50 rpm to 200 rpm, and preferably 80 to 150 rpm.

The stirring culture may be rotary stirring culture using a propeller or the like in a reactor. The stirring speed in the case of stirring by rotating is generally 50 rpm to 200 rpm, and preferably 80 to 150 rpm. In addition, stirring may be performed by wave-type shaking stirring or vertical movement of the stirring blade, but the stirring is not particularly limited.

The culture time in each step is not particularly limited, but is generally 6 hours to 14 days, preferably 12 hours to 7 days, more preferably 24 hours to 144 hours, and still more preferably 24 hours to 96 hours.

The titer of the manufactured adeno-associated virus can be measured by a conventional method known to those skilled in the art. For example, the cell culture solution after the culture is collected, the cells are disrupted by freeze-thaw, and then the supernatant is collected by centrifugation. The gDNA (genomic DNA) and the residual plasmid can be digested by adding MgCh and Benzonase to the collected supernatant to react. It is possible to measure the titer of AAV by performing ddPCR (Droplet Digital PCR) using the sample containing the adeno-associated virus obtained above as a ddPCR sample.

The present invention will be described in more detail with reference to the following examples, but the present invention is not limited to the examples.

### Examples

### [Materials and methods]

### <Cell culture>

Floating HEK293 cells (Thermo Fisher Scientific, R79007) were suspended in 12 mL of Expi29 Expression Medium (Thermo Fisher Scientific, A1435101) in a 1 × 10⁷ cells/mL, and cultured in 125 mL shaking flask for 24 hours. The culture solution was incubated under the conditions of 37°C and 8% CO₂ while being constantly stirred at 120 rpm.

### <Plasmid>

AAVpro (register trademark) Helper Free System (AAV5) (Takara Bio Inc., 6650) was used (Seq1, Seq2, Seq16).

The entire sequences of the used plasmids (Seq1 to Seq16, Seq20 to Seq23) are set forth in SEQ ID NOs: 1 to 16 and 20 to 23 in the sequence listing.

The AAVpro Helper Free System (AAV5) includes a pAAV-CMV Vector (seq16), a pRC5 Vector (seq2), and a pHelper Vector (seq1) as vector sequences.
pRC5 Vector: a plasmid containing the rep gene of AAV5 and the cap gene of AAV5.
pHelper Vector: a plasmid containing an adenovirus-derived E2A gene, E4 gene, and VA-RNA gene.
Seq1 (E2, E4, VA-RNA): a plasmid containing the pHelper Vector included in the AAVpro Helper Free System (AAV5) kit, and adenovirus-derived E2A gene, E4 gene, and VA-RNA gene.
Seq2 (REP, CAP): a plasmid containing the pRC Vector included in the AAVpro Helper Free System (AAV5) kit, the rep gene of AAV5, and the cap gene of AAV5.
Seq3 (ΔREP): a plasmid obtained by removing the rep gene coding region based on seq2 by inverse PCR and re-circularizing by self-ligation.
Seq4 (ΔCAP): a plasmid obtained by removing the cap gene coding region based on seq2 by inverse PCR and re-circularizing by self-ligation.
Seq5 (ΔE2): a plasmid obtained by removing the E2 gene coding region based on seq1 by inverse PCR and re-circularizing by self-ligation.
Seq6 (ΔE4): a plasmid obtained by removing the E4 gene coding region based on seq1 by inverse PCR and re-circularizing by self-ligation.
Seq7 (ΔVA-RNA): a plasmid obtained by removing the VA-RNA gene coding region based on seq1 by inverse PCR and re-circularizing by self-ligation.
Seq8 (REP): same sequence as seq4.
Seq9 (CAP): same sequence as seq3.
Seq10 (E2): a plasmid (GENEWIZ) obtained by synthesizing an E2 gene sequence and inserting the sequence into the EcoRV site of a pUC57 vector.
Seq11 (E4): a plasmid (GENEWIZ) obtained by synthesizing an E4 gene sequence and inserting the sequence into the EcoRV site of a pUC57 vector.
Seq12 (VA-RNA): a plasmid (GENEWIZ) obtained by synthesizing a VA-RNA gene sequence and inserting the sequence into the EcoRV site of a pUC57 vector.
Seq13 (E4ORF6): a plasmid obtained by amplifying a region of the E4ORF6 gene by PCR and inserting the amplified region into the ApaI/XbaI site of a pEBMulti-Neo vector (FUJIFILM Wako Pure Chemical Corporation).
Seq14 (VA-I): a plasmid obtained by removing the VA-RNA III gene coding region based on seq1 by inverse PCR and re-circularizing by self-ligation.
Seq15 (VA-II): a plasmid obtained by removing the VA-RNA I gene coding region based on seq1 by inverse PCR and re-circularizing by self-ligation.
Seq16: a vector plasmid containing the pAAV-CMV Vector included in the AAVpro Helper Free System (AAV5) kit, the MCS for inserting the CMV promoter and the target gene downstream of the CMV promoter, and two ITRs.
Seq20 (empty): a plasmid obtained by removing the E2 gene coding region, the E4 gene coding region, and the VA-RNA gene coding region based on seq1 by inverse PCR and re-circularizing by self-ligation.
Seq21 (Switch1-VAI): a plasmid (GENEWIZ) obtained by synthesizing a sequence in which an H1 promoter (RNA polymerase III promoter) and a VA-RNAI (VAI) gene are connected, and inserting the synthesized sequence into the EcoRV site of the pUC57 vector. The Switch1-VAI has a structure in which a Tet operator sequence is incorporated downstream of the RNA polymerase III promoter.
Seq22 (Switch2-VAI): a plasmid (GENEWIZ) obtained by synthesizing a sequence in which an U6 promoter (RNA polymerase III promoter) and a VA-RNAI (VAI) gene are connected, and inserting the synthesized sequence into the EcoRV site of the pUC57 vector. The Switch2-VAI has a structure in which a Tet operator sequence is incorporated upstream of the RNA polymerase III promoter.
Seq23 (tTS): a plasmid (GENEWIZ) obtained by inserting the synthesized, tetracycline-regulated transcriptional silencer (tTS) gene into the ClaI-XbaI site of the pLVSIN-CMV Hyg vector.
Seq38 (Switch-E4ORF6-Switch1-VAI-tTS): Switch1-VAI and tTS amplified by PCR were inserted into the plasmid (GENEWIZ) encoding the synthesized E4ORF6 gene sequence.
Seq39 (nRCp5): pRC5 Vector (seq2) was treated with a restriction enzyme with Bsp14071/BglII to prepare a rep-cap fragment. The rep-cap knock-in plasmid obtained by exchanging the rep-cap fragment between the Bsp14071/BglII sites of AAVS1 Safe Harbor cDNA/miRNA Donor Vector (pAAVS1D-PGK.MSC-Ef1α.copGFPpuro) (System Biosciences, GE603A-1), and re-circularizing by ligation.
Seq40 (p5RCp5): a plasmid obtained by inserting the synthesized p5 promoter sequence into Seq39. Seq40 has p5 promoter sequences before and after the rep-cap gene.
Seq41 (E2-GOI): a plasmid (GENEWIZ) obtained by synthesizing the gene sequence having a GFP gene and a puromycin-resistant gene flanked by the E2 gene and AAV ITR sequence, and a piggyBac transposon sequence, and inserting the synthesized gene sequence into the EcoRV site of the pUC57 vector.
Seq42: a plasmid (GENEWIZ) obtained by synthesizing the piggyBac transposase gene sequence and inserting the gene sequence into the EcoRV site of the pUC57 vector.
Seq43 (all-in-one): a plasmid obtained by integrating a REP gene, a CAP gene, an E2 gene, Switch-E4ORF6, Switch1-VAI, and a tTS prepared by synthesis. The plasmid map of seq43 is shown in Fig. 11.

### <Preparation of plasmid by PCR reaction>

Primer 1 and Primer 2 (final concentration of 0.2 µmol/L), and PrimeSTAR Max Premix (1×) were mixed with 10 pg of the template DNA, and the total amount was prepared to 50 µL using nuclease-free water (Thermo Fisher Scientific, 10977015). The primer1 and primer2 used in the above-described plasmid preparation are shown below. The base sequences of seq24 to seq37 are set forth in SEQ ID NOs: 24 to 37 in the sequence listing.

A plasmid of seq2 was used as a template to prepare seq3 and seq4 by inverse PCR, and seq1 was used as a template to prepare seq5 to seq7, seq14, seq15, and seq20 by inverse PCR. The following shows (prepared plasmid): (primer set used in inverse PCR).
Seq3: seq24 and seq25
Seq4: seq26 and seq27
Seq5: seq28 and seq29
Seq6: seq30 and seq31
Seq7: seq32 and seq33
Seq14: seq34 and seq35
Seq15: seq36 and seq37
Seq20: seq29 and seq32

The prepared sample was reacted at 98°C for 10 seconds, 55°C for 15 seconds, and 72°C for 5 seconds per kbp for 30 cycles. The reactants were cleaved with the restriction enzyme NotI (TOYOBO CO., LTD., NOT-111X), and the plasmid circularized with T4 DNA Ligase (Takara Bio Inc., 2011A) was then amplified with E. Coli (Takara Bio Inc., 9128).

### <Gene transfer method and evaluation of cell disorder>

36 µL of Polyethylenimine (PEI) (PolyPlus-transfection SAS, 115-0015) and an equal amount of the plasmids of the combination described below were mixed, and a total amount of 18 µg of the mixture was suspended in 1.8 mL of Expi29 Expression Medium and allowed to stand for 15 minutes. Then, the above-mentioned reagent was added to the cultured cells (culture solution volume was 12 mL) seeded at a cell concentration of 1 × 10⁷ cells/mL on the previous day, and the cells were incubated under the conditions of 37°C and 8% CO₂.

The disorder property of the transferred cells was evaluated by the viability of the cells by trypan blue staining on Day4, Day7, and Day11 after the gene transfer. The calculation was performed with the cytotoxic cell rate (%) = 100 - (cell viability).

### <Western blot>

Cells were collected by centrifugation and lysed with a RIPA Buffer. Sample Buffer (nacalai tesque, 09499-14) was added to the cell solution and the mixture was heated at 100°C for 5 minutes to prepare a sample. The protein in the sample was size-separated using a sodium dodecyl sulfate (SDS) gel electrophoresis device (ATTO CORPORATION, 2321670), and then transferred to a membrane using a semi-dry blotting device (ATTO CORPORATION, 2322490). The membrane after transfer was blocked with Blocking One (nacalai tesque, 03953-95) for 30 minutes, then reacted with an anti-REP antibody (OriGene Technologies) diluted 70-fold with a blocking solution, an anti-VP antibody (PROGEN Biotechnik GmbH) that reacts with a capsid structural protein, and an anti-β-Actin antibody (Abcam) at room temperature for 3 hours. Next, the reactant was reacted with anti-mouse IgG-HRP (Cytiva), as a secondary antibody, diluted 10,000-fold with a blocking solution at room temperature for 2 hours, and then the REP protein and the capsid structural proteins (VP1, VP2, VP3) were detected with a SuperSignal West Femto Maximum Sensitivity Substrate (Thermo Fisher Scientific, 34095). WSE-6100 LuminoGraphI (ATTO CORPORATION, 2006100) was used for imaging. Quantitative evaluation was performed using ImageJ for the analysis of the acquired image.

### <Measurement of AAV titer>

The HEK cell culture medium, which had been cultured for 72 hours after the gene transfer under the conditions of 37°C and 8% CO₂, was collected, and the cells were disrupted by freezing and thawing at -80°C. Then, the supernatant was collected by centrifugation at 13,800 ×g, 2 mmol/L (final concentration) MgCl₂, and 25 U/mL Benzonase was added thereto, and a reaction was carried out at 37°C for 2 hours to digest gDNA (genomic DNA) and residual plasmid. The sample after the reaction was incubated at 95°C for 15 minutes, 70°C for 3 minutes, 40°C for 3 minutes, and 20°C for 3 minutes in this order, to inactivate Benzonase, and the reactant was used as a ddPCR (Droplet Digital PCR) sample. The ddPCR sample was diluted 50-fold with a TE buffer (pH 8.0, containing 0.05% Pluronic F-68 and 10 µg/mL bovine thymus DNA). 10 µL of ddPCRtm Supermix for Probes (no dUTP) (Bio-Rad Laboratories, Inc.), 2 µL of diluted ddPCR sample, primers (seq17, seq18, final concentration of 900 nmol/L) and probe (seq19, final concentration of 250 nmol/L), nuclease free water (Thermo Fisher Scientific, 10977015) was mixed in a tube on ice, the total liquid volume was adjusted to 22 µL, and ddPCR was performed. The sequences of seq17, seq18, and seq19 are set forth in SEQ ID NOs: 17 to 19 in the sequence listing.

In ddPCR, droplets were formed from the prepared solution using a droplet forming apparatus, and subsequently incubated at 95°C for 10 minutes, 94°C for 30 seconds, and 55°C in this order for 40 cycles, and then incubated at 98°C for 10 minutes. Measurement was performed using a droplet reader, and the AAV genomic titer (vg/mL) was calculated.

### <Establishment of cell strain>

36 µL of Polyethylenimine (PEI) (PolyPlus-transfection SAS, 115-0015) and an equal amount of the plasmids of the combination described below were mixed, and a total amount of 18 µg of the mixture was suspended in 1.8 mL of Expi29 Expression Medium and allowed to stand for 15 minutes. Then, the above-mentioned reagent was added to the cultured cells (culture solution volume was 12 mL) seeded at a cell concentration of 1 × 10⁷ cells/mL on the previous day, and the cells were incubated under the conditions of 37°C and 8% CO₂. The gene-transferred cells were maintained and cultured for 3 weeks, and RFP or GFP fluorescence-positive cells were isolated as single cells using flow cytometry. The isolated cells were suspended in DMEM containing 10% bovine fetal serum and cultured on a 6-well plate under the conditions of 37°C and 8% CO₂ for 1 month to establish a cell strain.

### [Result]

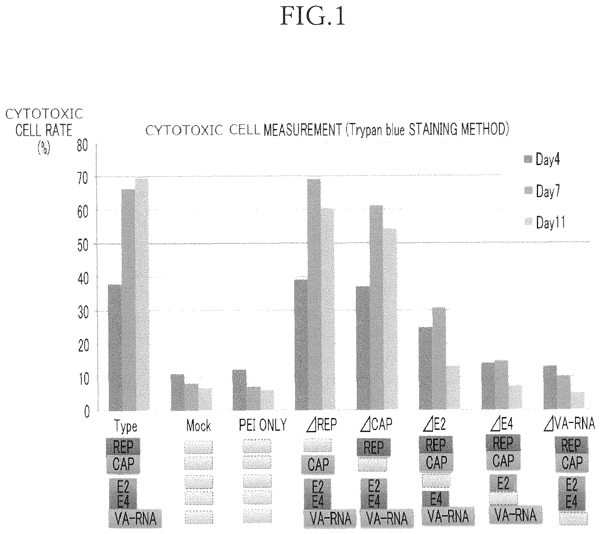

Fig. 1 shows the results of evaluating cytotoxicity by transferring genes into HEK293 cells in the following combinations.
Type: seq1, seq2
Mock: seq20
PEI only: no gene
ΔREP: seq1, seq3
ΔCAP: seq1, seq4
ΔE2: seq2, seq5
ΔE4: seq2, seq6
ΔVA-RNA: seq2, seq7

Cytotoxic rate (hereinafter, the cytotoxic rates of Day4, Day7, and Day11 are shown from the left).
Type: 38.0%, 66.6%, 69.6%
Mock: 11.0%, 8.3%, 6.9%
PEI only: 12.4%, 7.1%, 6.3%
ΔREP: 39.1%, 69.2%, 60.4%
ΔCAP: 37.0%, 61.2%, 54.3%
ΔE2: 24.7%, 30.6%, 13.3%
ΔE4: 14.0%, 14.8%, 7.4%
ΔVA-RNA: 13.1%, 10.2%, 5.2%

From the results shown in Fig. 1, in Day11, cell disorder was reduced by removing the E2 gene, the E4 gene, and the VA-RNA gene.

Furthermore, from the results shown in Fig. 1, it was confirmed that the absence of the E4 gene and the VA-RNA gene can avoid cell disorder.

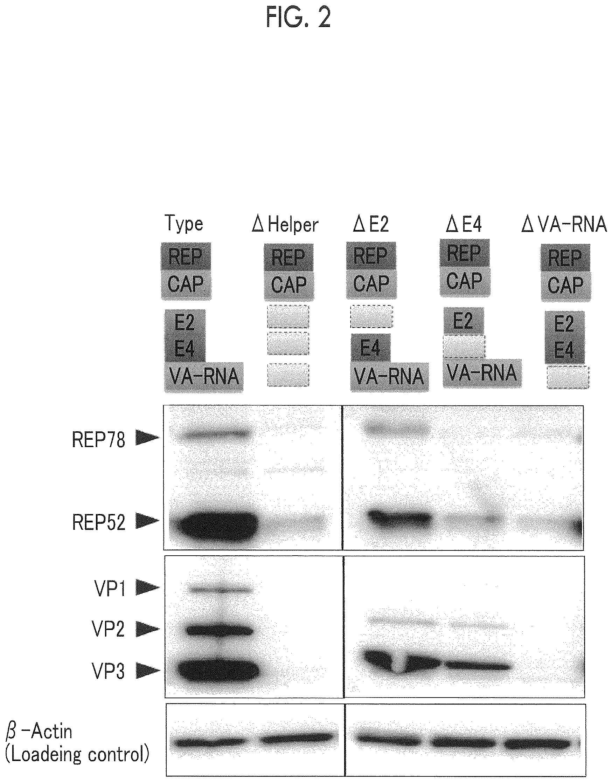

Fig. 2 shows the results of evaluating protein expression by transferring genes into HEK293 cells in the following combinations.
Type: seq1, seq2
ΔHelper: seq2
ΔE2: seq2, seq5
ΔE4: seq2, seq6
ΔVA-RNA: seq2, seq7

From the results of Fig. 2 and Table 1 in which the bands of Fig. 2 were quantified, it was confirmed that the expression of the REP protein and the capsid structural protein was suppressed by removing the helper gene. It was confirmed that the expression of REP (with cell disorder) can be controlled in a manner dependent on the E4 gene and the VA-RNA gene among the helper genes.

**[Table 1]**

| | | | | | |
|---|---|---|---|---|---|
| Expression level ratio to Type (Various proteins/Loading control) | | | | | |

| | REP78 | REP52 | VP1 | VP2 | VP3 |
|---|---|---|---|---|---|
| Type | 1 | 1 | 1 | 1 | 1 |
| ΔHelper | 0.17 | 0.19 | 0.08 | 0.05 | 0.11 |
| ΔE2 | 0.49 | 0.74 | 0.10 | 0.14 | 0.89 |
| ΔE4 | 0.18 | 0.19 | 0.10 | 0.10 | 0.31 |
| ΔVA-RNA | 0.22 | 0.22 | 0.11 | 0.08 | 0.12 |

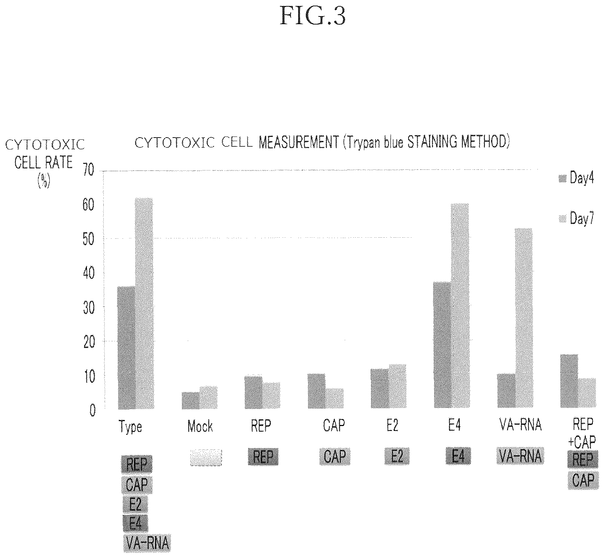

Fig. 3 shows the results of evaluation of cytotoxicity by transferring genes into HEK293 cells in the following combinations.
Type: seq1, seq2
Mock: seq20
REP: seq8
CAP: seq9
E2: seq10
E4: seq11
VA-RNA: seq12
REP + CAP: seq2

Cytotoxic cell rate (hereinafter, the cytotoxic rate of Day4 and Day7 is shown from the left)
Type: 36.0%, 62.1%
Mock: 5.2%, 6.7%
REP: 9.7%, 7.9%
CAP: 10.4%, 6.1%
E2: 11.6%, 12.9%
E4: 36.9%, 60.1%
VA-RNA: 9.9%, 52.7%
REP + CAP: 15.6%, 8.8%

From the results shown in Fig. 3, it was confirmed that the E4 gene and the VA-RNA gene have cytotoxicity even in a case of being transferred singly, and their expression is required to control.

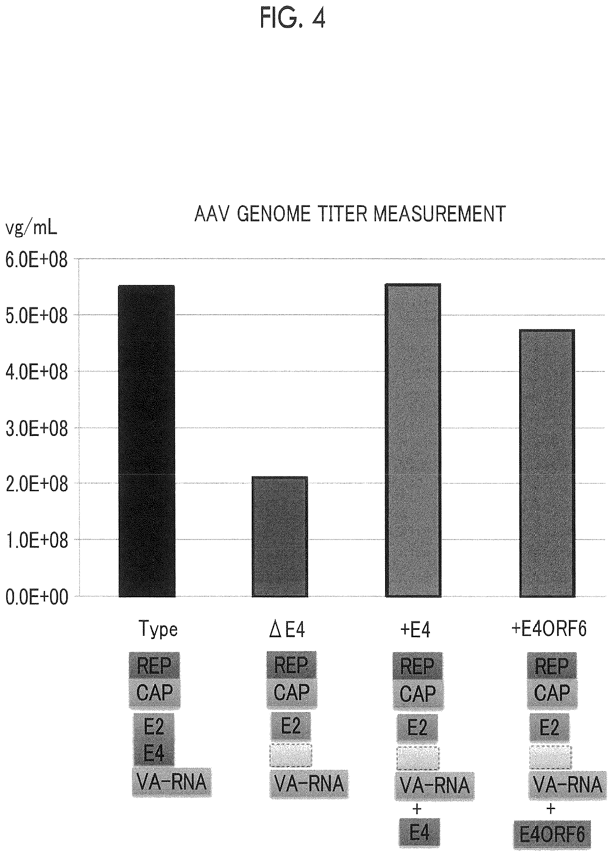

Fig. 4 shows the results of evaluation of AAV titer by transferring genes into HEK293 cells in the following combinations.
Type: seq1, seq2, seq16
-E4: seq2, seq6, seq16
+E4: seq2, seq6, seq11, seq16
+E4ORF6: seq2, seq6, seq13, seq16

### Virus amount (vg/mL)

Type: 5.5 × 10⁸
ΔE4: 2.1 × 10⁸
+E4: 5.5 × 10⁸
+E4ORF6: 4.7 × 10⁸

From the results shown in Fig. 4, it was confirmed that the E4ORF6 gene alone produced the virus to the same extent as in the case where the E4 gene was transferred, and the function of the E4 gene could be substituted.

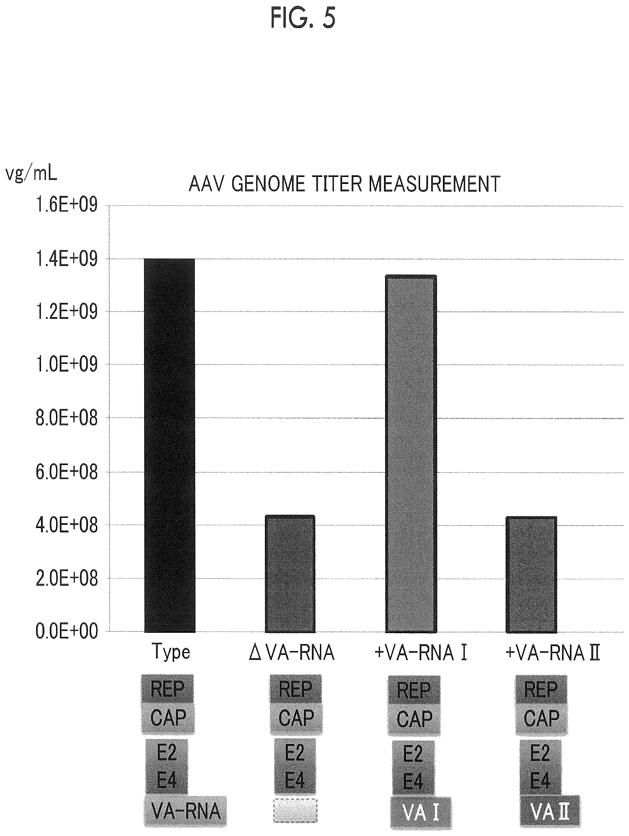

Fig. 5 shows the results of evaluation of AAV titer by transferring genes into HEK293 cells in the following combination.
Type: seq1, seq2, seq16
-VA-RNA: seq2, seq7, seq16
+VA-RNAI: seq2, seq14, seq16
+VA-RNAII: seq2, seq15, seq16

### Virus amount (vg/mL)

Type: 1.4 × 10⁹
ΔVA-RNA: 4.3 × 10⁹
+VA-RNAI: 1.3 × 10⁹
+VA-RNAII: 4.3 × 10⁹

From the results shown in Fig. 5, it was confirmed that the VA-RNAI (VAI) gene alone was expressed to the same extent as in the case where the VA-RNA gene was transferred, and the function of the VA-RNA gene could be substituted.

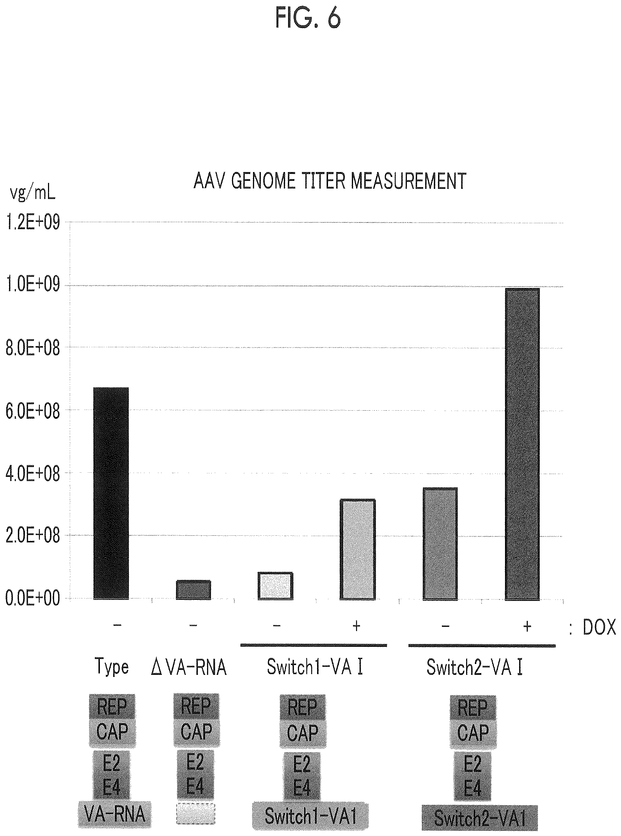

Fig. 6 shows the results of evaluation of AAV titer by transferring genes into HEK293 cells in the following combination. In addition, doxycycline (DOX) having a final concentration of 0.5 µg/mL was added to the "Switch1-VAI + DOX" and "Switch2-VAI + DOX" samples after gene transfer. The AAV genomic titer of HEK293 cells cultured for 72 hours after the addition of DOX was evaluated.
Type: seq1, seq2, seq16
ΔVA-RNA: seq2, seq7, seq16
Switch1-VAI: seq2, seq7, seq16, seq21, seq23
Switch2-VAI: seq2, seq7, seq16, seq22, seq23

### Virus amount (vg/mL)

Type: 6.7 × 10⁸
-VA-RNA: 5.6 × 10⁷
Switch1-VAI + DOX: 8.2 × 10⁷
Switch1-VAI - DOX: 3.2 × 10⁸
Switch2-VAI + DOX: 3.5 × 10⁸
Switch2-VAI - DOX: 9.9 × 10⁸

From the results shown in Fig. 6, it was confirmed that the addition of DOX improved the AAV production amount. It was confirmed that the use of Switch1 can suppress AAV production in a case where DOX was not added, as compared with Switch2. In addition, it was confirmed that the production of AAV was increased by using Switch2 as compared with Switch1. It was found that by using VA-1 with Switch2, the expression was increased by adding DOX even as compared with Type in which Switch was not added. From the viewpoint that AAV production can be suppressed in a case where DOX is not added, Switch1 is more preferable.

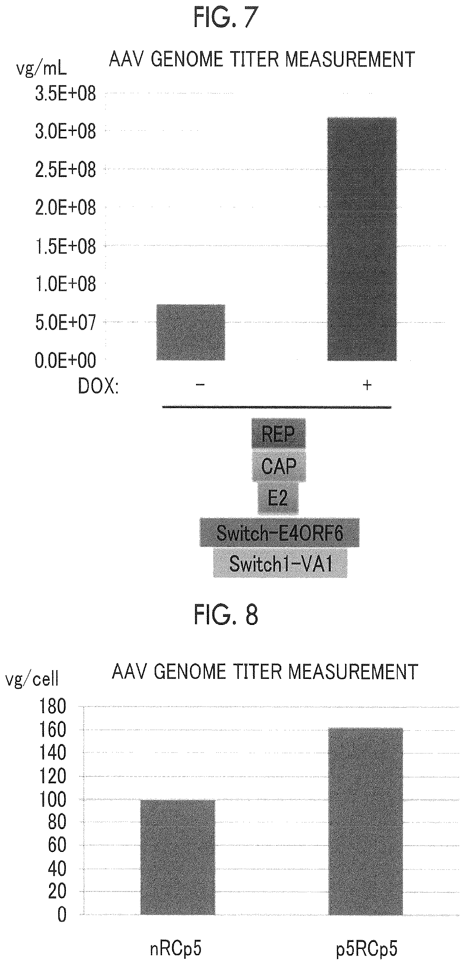

Fig. 7 shows the results of evaluation of AAV titer by transferring genes into HEK293 cells with a combination of seq2, seq10, seq16, and seq38. In addition, doxycycline (DOX) having a final concentration of 0.5 µg/mL was added to the "+DOX" sample after the gene was transferred. The AAV genomic titer of HEK293 cells cultured for 72 hours after the addition of DOX was evaluated.

### Virus amount (vg/mL)

-DOX: 7.4 × 10⁷
+DOX: 3.2 × 10⁸

A stable expression strain was established by transferring seq39 or seq40 and Cas9 SmartNuclease AAVS1-gRNA Targeting Vector (System Biosciences, CAS601A-1) into HEK293 cells. Fig. 8 shows the results of evaluation of AAV titers by transferring plasmids into established cells in the following combinations.
nRCp5: rep-cap gene-inserted cell, seq1, seq20, seq16
p5RCp5: rep-cap gene-inserted cells in which p5 is added before and after the rep-ca-gene, seq1, seq20, seq16

The rep-cap strain established above was cultured for one month. Then, it was confirmed that the introduced sequence was integrated into the chromosome of the cell by confirming that PCR amplification is performed using a chromosome extracted from a cell as a template, by a droplet digital PCR method using a primer and a probe designed to be amplified only in the presence of the vector sequence.

### Virus amount (vg/cell)

nRCp5: 9.9 × 10¹
p5RCp5: 1.6 × 10²

From the results shown in Fig. 8, it was confirmed that a cell strain into which the rep-cap gene whose expression is not controlled could be inserted can be established, and further AAV could be produced by transferring the E2 gene, the E4 gene, the VA-RNA gene, and the GOI gene later.

NGS analysis was carried out on p5RCp5, and by reading a sequence of cells, it was confirmed that the rep gene and the cap gene were integrated into the chromosome.

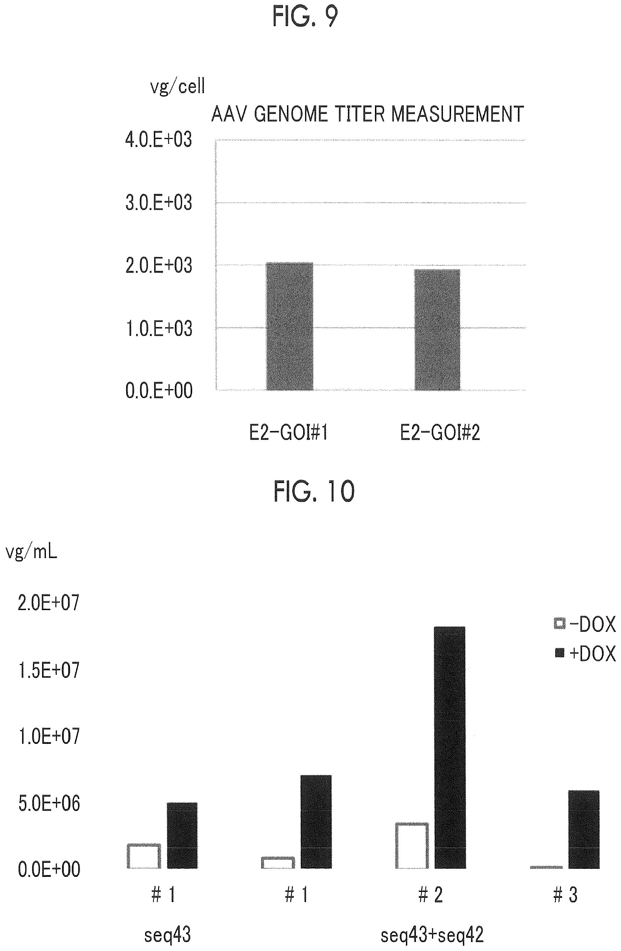

Two stable expression strains were established by transferring seq41 and seq42 into HEK293 cells (E2-GOI #1 and #2). Fig. 9 shows the results of evaluation of AAV titers by transferring plasmids in a combination of seq2 and seq5 into established cells.

The two E2-GOI strains established above were cultured for one month. Then, it was confirmed that the introduced sequence was integrated into the chromosome of the cell by confirming that PCR amplification is performed using a chromosome extracted from a cell as a template, by a droplet digital PCR method using a primer and a probe designed to be amplified only in the presence of the vector sequence.

### Virus amount (vg/cell)

E2-GOI #1: 2.1 × 10³
E2-GOI #2: 1.9 × 10³

From the results shown in Fig. 9, it was confirmed that a cell strain into which the E2 gene-GOI gene was inserted could be established, and further AAV could be produced by transferring the REP gene, the CAP gene, the E4 gene, and the VA-RNA gene later.

Cell strains were established by seq43 alone or by co-transferring seq43 and seq42, respectively. Doxycycline (DOX) having a final concentration of 0.5 µg/mL was added to the "+DOX" sample for the strain 1.5 months after the establishment. The AAV genomic titer of cells cultured for 72 hours after the addition of DOX was evaluated. The results are shown in Fig. 10.

### Virus amount (vg/mL)

Seq43 strain
#1 -DOX: 1.8 × 10⁶
#1 +DOX: 5.0 × 10⁶
Seq43 + Seq42 strain
#1 -DOX: 8.7 × 10⁵
#1 +DOX: 7.1 × 10⁶
#2 -DOX: 3.5 × 10⁶
#2 +DOX: 1.8 × 10⁷
#3 -DOX: 2.1 × 10⁵
#3 +DOX: 5.9 × 10⁶
[Sequence list] International application 20F01635W1JP22005335_1.app based on International Patent Cooperation Treaty

## Claims

1. A kit comprising:
a first vector containing one or more virus helper genes, in which at least one of the virus helper genes is under a control of a promoter capable of regulating expression; and
a second vector containing one or more adeno-associated virus genes, in which at least one of the adeno-associated virus genes is not under a control of a promoter regulating expression by a drug.

2. The kit according to claim 1,
wherein the promoter capable of regulating expression in the first vector is a promoter capable of regulating expression by a drug.

3. The kit according to claim 1 or 2,
wherein the virus helper gene contained in the first vector is derived from adenovirus.

4. The kit according to any one of claims 1 to 3,
wherein among the virus helper genes contained in the first vector, an E4 gene and a VA-RNA gene are under a control of a promoter capable of regulating expression.

5. The kit according to any one of claims 1 to 4,
wherein all of the virus helper genes contained in the first vector are under a control of a promoter capable of regulating expression.

6. The kit according to any one of claims 1 to 5,
wherein the virus helper genes contained in the first vector are an E2 gene, an E4 gene, and a VA-RNA gene.

7. The kit according to claim 6,
wherein the E4 gene is an E4ORF6 gene.

8. The kit according to claim 6 or 7,
wherein the VA-RNA gene is a VA-RNAI gene.

9. The kit according to any one of claims 1 to 8,
wherein in the second vector, all of the adeno-associated virus genes are not under a control of a promoter regulating expression by a drug.

10. The kit according to any one of claims 1 to 9,
wherein in the second vector, any one of the adeno-associated virus genes, which is not under a control of a promoter regulating expression by a drug is under a control of a promoter that is naturally carried by the adeno-associated virus gene.

11. The kit according to any one of claims 1 to 10,
wherein the adeno-associated virus genes contained in the second vector are a rep gene and a cap gene.

12. The kit according to any one of claims 1 to 11,
wherein among the adeno-associated virus genes contained in the second vector, the adeno-associated virus gene that is not under a control of a promoter regulating expression by a drug is a rep gene.

13. A cell comprising:
the first vector and the second vector which are included in the kit according to any one of claims 1 to 12.

14. The cell according to claim 13,
wherein the cell is a human cell.

15. The cell according to claim 13 or 14,
wherein the first vector and the second vector are contained in a chromosome.

16. An integration vector comprising in a connected state:
a first vector containing one or more virus helper genes, in which at least one of the virus helper genes is under a control of a promoter capable of regulating expression; and
a second vector containing one or more adeno-associated virus genes, in which at least one of the adeno-associated virus genes is not under a control of a promoter regulating expression by a drug.

17. The integration vector according to claim 16, further comprising:
a third vector containing a gene for desired treatment or prevention.

18. A cell comprising:
the integration vector according to claim 16 or 17.

19. The cell according to claim 18,
wherein the cell is a human cell.

20. The cell according to claim 18 or 19,
wherein the integration vector is contained in a chromosome.

21. A manufacturing method of a cell for producing an adeno-associated virus, comprising:
transferring, into a cell, a first vector containing one or more virus helper genes, in which at least one of the virus helper genes is under a control of a promoter capable of regulating expression, and a second vector containing one or more adeno-associated virus genes, in which at least one of the adeno-associated virus genes is not under a control of a promoter regulating expression.

22. A manufacturing method of an adeno-associated virus, comprising:
a step of culturing cells that contain a first vector containing one or more virus helper genes, in which at least one of the virus helper genes is under a control of a promoter capable of regulating expression, a second vector containing one or more adeno-associated virus genes, in which at least one of the adeno-associated virus genes is not under a control of a promoter regulating expression, and as necessary, a third vector containing a gene for desired treatment or prevention; and
a step of expressing the virus helper gene under the control of the promoter capable of regulating expression by activating the promoter capable of regulating expression.

23. A manufacturing method of an adeno-associated virus, comprising:
a first step of transferring a part of a virus helper genes and a part of an adeno-associated virus genes into a cell;
a second step of culturing and proliferating the cell obtained in the first step;
a third step of transferring a virus helper gene not contained in the cell obtained in the first step, an adeno-associated virus gene not contained in the cell obtained in the first step, and as necessary, a gene for desired treatment or prevention, into the cells obtained in the second step; and
a fourth step of culturing the cells obtained in the third step.

24. The manufacturing method of an adeno-associated virus according to claim 23,
wherein the virus helper gene transferred into the cell in the first step is an E2 gene, the adeno-associated virus gene transferred into the cell in the first step is a rep gene, the virus helper genes transferred into the cells in the third step are an E4 gene and a VA-RNA gene, and the adeno-associated virus gene transferred into the cells in the third step is a cap gene.

25. A manufacturing method of an adeno-associated virus, comprising:
a first step of transferring a part of a virus helper gene, an adeno-associated virus gene, and as necessary, a gene for desired treatment or prevention, into a cell;
a second step of culturing and proliferating the cell obtained in the first step,
a third step of transferring a virus helper gene not contained in the cell in the first step into the cells obtained in the second step; and
a fourth step of culturing the cells obtained in the third step.

26. The manufacturing method of an adeno-associated virus according to claim 25,
wherein the virus helper gene transferred into the cell in the first step is an E2 gene, the adeno-associated virus genes transferred into the cell in the first step are a rep gene and a cap gene, and the virus helper genes transferred into the cells in the third step are an E4 gene and a VA-RNA gene.

27. A manufacturing method of an adeno-associated virus, comprising:
a first step of transferring an adeno-associated virus gene into a cell;
a second step of culturing and proliferating the cell obtained in the first step,
a third step of transferring a virus helper gene and as necessary, a gene for desired treatment or prevention into the cells obtained in the second step; and
a fourth step of culturing the cells obtained in the third step.

28. The manufacturing method of an adeno-associated virus according to claim 27,
wherein the adeno-associated virus genes transferred into the cell in the first step are a rep gene and a cap gene, and the virus helper genes transferred into the cells in the third step are an E2 gene, an E4 gene, and a VA-RNA gene.

29. A manufacturing method of an adeno-associated virus, comprising:
a first step of transferring a part of a virus helper gene and as necessary, a gene for desired treatment or prevention, into a cell;
a second step of culturing and proliferating the cell obtained in the first step,
a third step of transferring a virus helper gene not contained in the cell in the first step and an adeno-associated virus gene into the cells obtained in the second step; and
a fourth step of culturing the cells obtained in the third step.

30. The manufacturing method of an adeno-associated virus according to claim 29,
wherein the virus helper gene transferred into the cell in the first step is an E2 gene, the virus helper genes transferred into the cells in the third step are an E4 gene and a VA-RNA gene, and the adeno-associated virus genes transferred into the cells in the third step is a rep gene and a cap gene.
